# DEMANDE DE BREVET EUROPEEN

(11) **EP 1 300 109 A1**
(43) Date de publication de la demande: **09.04.2003**
(21) Numéro de dépôt: 02292423.7
(22) Date de dépôt: 02.10.2002
(51) Int. Cl.: A61B 3/13, G02B 21/00, G02B 21/06

(54) **Microscope à lampe à fente pour la microchirurgie oculaire**

(30) Priorité: 02.10.2001 FR 0112681
(71) Demandeur: Ducournau, Didier, 44000 Nantes (FR)
(72) Inventeur: Ducournau, Didier, 44000 Nantes (FR)
(74) Mandataire: Catherine, Alain

(57) **Abrégé**

L'invention concerne un microscope pour la microchirurgie oculaire comprenant :
- un objectif principal (2),
- au moins un système oculaire (3), et
- des moyens d'éclairage à fente,
l'image de la fente (16) étant projetée sur le fond d'oeil (10) au travers d'un prisme (21) placé à proximité de la direction d'observation du microscope.

Le prisme (21) est relié à la source lumineuse (14) par un faisceau (15) de fibres optiques, la source (14) étant indépendante du microscope.

## Description

L'invention concerne les microscopes utilisés pour la microchirurgie oculaire.

Les microscopes opératoires comportent généralement :
- un système d'oculaires pour l'opérateur et, généralement, pour son assistant,
- un système de grossissement adaptable, le plus souvent par zoom,
- un système de mise au point, les focales les plus fréquemment employées étant de l'ordre de 175 ou 200 millimètres,
- un système d'éclairage coaxial, généralement fourni par une source externe au corps du microscope et transmis par des fibres optiques.

De tels microscopes permettent l'analyse des surfaces de la cornée et du segment antérieur de l'oeil (iris, face antérieur de l'iris et cristallin). La visualisation des structures plus postérieures (vitré, rétine) est impossible sans autre système optique du fait des dioptres cornéens et cristalliniens. Pour compenser l'effet de ces dioptres, l'opérateur devra soit placer une lentille sur la cornée, soit utiliser un système de lentilles à distance de la cornée (un des systèmes les plus utilisés actuellement est le BIOM, système de vision panoramique, fixé à la partie inférieure du microscope). Ces systèmes de lentilles ne permettent cependant pas l'examen des structures vitréennes et rétiniennes sous l'éclairage coaxial du microscope à cause des reflets engendrés par cet éclairage. C'est pourquoi, la plupart des opérateurs choisissent d'utiliser comme source d'éclairage des fibres illuminantes intraoculaires. Une autre façon de procéder est d'utiliser un éclairage par fente lumineuse placée à un angle réduit par rapport à l'axe d'observation (de 0 à 30 degrés) et permettant de passer au travers de la pupille sans engendrer les reflets d'un éclairage coaxial.

Ces systèmes à lampe à fente équipent les biomicroscopes qui permettent aux ophtalmologistes d'examiner leur patients dans leurs locaux de consultation.

La fixation d'une lampe à fente au microscope opératoire a été réalisée et commercialisée depuis plus de 30 ans. La fente lumineuse est issue d'une source éclairante soit intégrée au système, soit déportée et reliée au système par une fibre optique. Le rayon lumineux traverse une fente de largeur réglable. La fente lumineuse ainsi obtenue est déviée par un miroir placé à proximité de l'axe d'observation et orienté de façon à ce qu'elle frappe le milieu du champ d'observation à la focale désirée. Ce système est monté sur un support fixé à la partie inférieure du microscope. Ce support permet de faire varier l'angle d'éclairage de la fente par rapport à l'axe d'observation. En effet, un angle de débattement était jusqu'à présent considéré comme nécessaire pour permettre une observation optimale.

L'utilisation per-opératoire d'un éclairage par lampe à fente fixée au microscope présente de nombreux avantages :
- libération de la main gauche chargée de tenir la fibre illuminante intraoculaire,
- coupe optique des tissus transparents (cornée, cristallin, vitré...),
- diminution des risque de phototoxicité de la fibre endoculaire (liée au carré de la distance entre la fibre et la rétine).

L'utilisation de ces systèmes à lampe à fente fixés au microscope a été cependant abandonnée par la majorité des opérateurs pour deux raisons principales :
1°) encombrement : les systèmes à lampe à fente actuellement commercialisés sont volumineux et gênants pour les chirurgiens qui n'utilisent pas ce système d'éclairage. Ils posent de gros problèmes de stérilisation. Ils peuvent toucher le champ opératoire.
2°) incompatibilité avec certains systèmes optiques grand champ fixés à la partie inférieure du microscope (BIOM par exemple).

L'objectif de l'invention est de proposer un système d'éclairage par lampe à fente n'entraînant pas les inconvénients des systèmes actuels.

A cet effet, l'invention concerne un microscope opératoire équipé d'un système d'éclairage par lampe à fente ne dépassant pas de plus de 15 à 20 mm la partie la plus inférieure de l'objectif principal.

L'inventeur a constaté que, contrairement aux idées répandues, il est possible d'utiliser une lampe à fente sans faire varier son angle par rapport à l'angle d'observation, dans pratiquement 100 % des cas. Un angle fixe, compris entre 2 et 10 degrés, donne satisfaction, le meilleur éclairage étant donné avec un angle de 4 à 8 degrés. La suppression du bras de translation permet ainsi d'avoir un système plus compact et moins encombrant. En effet, le caractère fixe du miroir ou du prisme, servant à dévier la fente lumineuse pour lui donner un axe proche de l'axe d'observation, permet d'envisager de le placer, soit à l'intérieur même du corps du microscope, soit très proche de l'objectif principal dudit microscope, sans dépasser les 20 mm d'encombrement.

La limitation de cet encombrement est obtenue en dissociant la source du microscope lui-même et en les reliant par un faisceau de fibres.

A cet effet, l'invention concerne un microscope pour la microchirurgie oculaire comprenant :
- un objectif principal,
- au moins un système oculaire, et
- des moyens d'éclairage à fente,
l'image de la fente étant projetée sur le fond d'oeil au travers d'un prisme placé à proximité de la direction d'observation du microscope.

Selon l'invention, le prisme est relié à la source lumineuse par un faisceau de fibres optiques, la source étant indépendante du microscope.

Selon les modes de réalisation, ce prisme comporte un réflecteur 100 % et peut alors être un miroir ou il comporte un réflecteur partiel permettant une observation au travers de ce composant.

Dans différents modes de réalisation préférés, présentant chacun des avantages spécifiques, le microscope opératoire de l'invention comportera les caractéristiques suivantes :
- les moyens d'éclairage à fente produisent un faisceau d'éclairage incliné par rapport à la direction d'observation d'un angle fixe,
- les moyens d'éclairage à fente produisent un faisceau d'éclairage incliné par rapport à la direction d'observation d'un angle compris entre 2 et 10 degrés,
- le prisme est placé en amont de l'objectif principal,
- le prisme est placé en amont d'une partie au moins des éléments optiques de l'un des systèmes oculaires,
- le prisme est placé en aval de l'objectif principal grâce à une fixation rapportée autour du corps de l'objectif principal,
- l'éclairage de la lampe à fente est indépendant d'un éclairage coaxial du microscope,
- la largeur de la fente est réglable,
- la fente est portée par un barillet en comportant plusieurs de largeurs différentes permettant au praticien de choisir l'une d'entre elles.

Différents modes de réalisation de l'invention seront décrits en détail en référence aux dessins dans lesquels :
- la figure 1 est une vue de côté d'un premier mode de réalisation de l'invention ;
- la figure 2 est une vue de face d'un deuxième mode de réalisation de l'invention ;
- la figure 3 est une vue de face d'un troisième mode de réalisation.

Ces différents modes de réalisation de l'invention sont représentés avec un microscope binoculaire 1 comportant un objectif principal 2 et deux systèmes oculaires 3 et 3'.

Ces systèmes oculaires comportent chacun deux lentilles 4 et 5 et un ensemble de prismes 6.

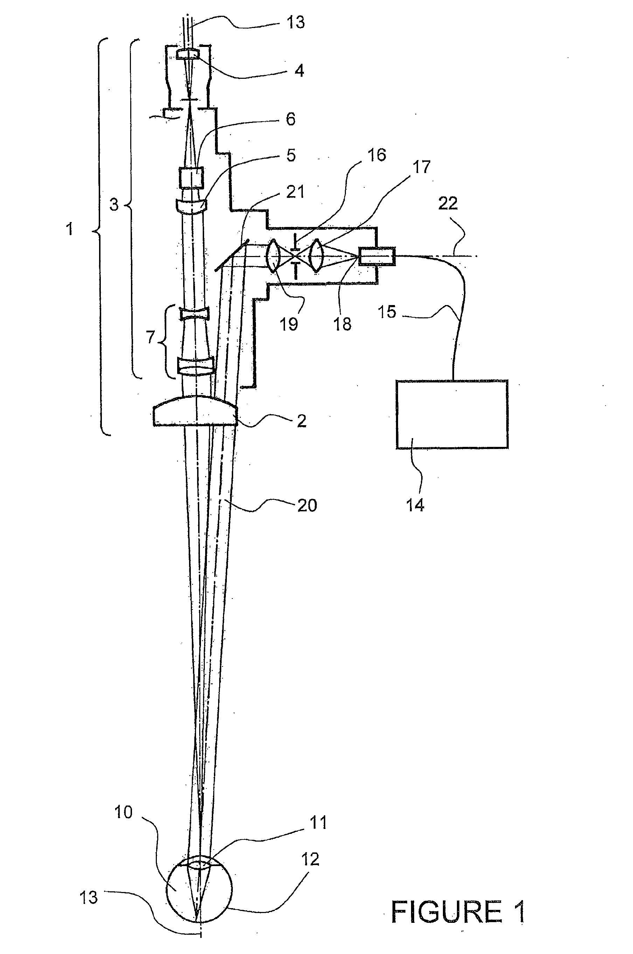

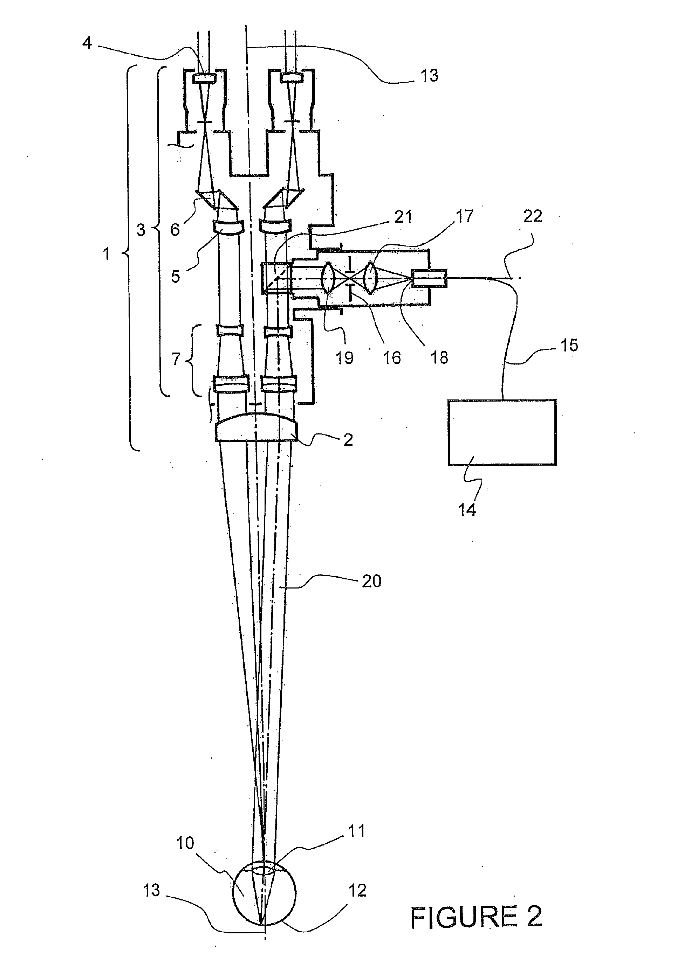

Certains de ces microscopes représentés sur les figures 1 et 2 comportent un ensemble optique 7 permettant de faire varier le grossissement de l'appareil.

Ce microscope permet l'observation de l'oeil 10 que l'on a représenté traditionnellement avec son cristallin 11 et sa rétine 12, la direction d'observation du microscope et l'axe 13 de celui-ci.

Ce microscope est équipé d'une source lumineuse 14 généralement alimentée par le secteur électrique. Cette source 14 constitue un bloc indépendant relié au microscope par un faisceau de fibres optiques 15. Le microscope comporte une fente 16 qui est conjuguée par un condenseur 17 à l'extrémité 18 du faisceau de fibres optiques.

Un collimateur 19 produit, à partir du flux lumineux reçu et émis par la fente 16, un faisceau lumineux parallèle 20 de section rectangulaire.

Dans le premier mode de réalisation représenté sur la figure 1, le faisceau qui a une direction initiale 22 est perpendiculaire à la direction 13 d'observation du microscope et dirigé vers l'oeil par un cube réflecteur 21 au travers de l'objectif principal 2. Le prisme ou le cube réflecteur 21 peut alors être un simple miroir.

Dans le deuxième mode de réalisation illustré par la figure 2, le faisceau traverse également les moyens optiques 7 de contrôle du grossissement du microscope.

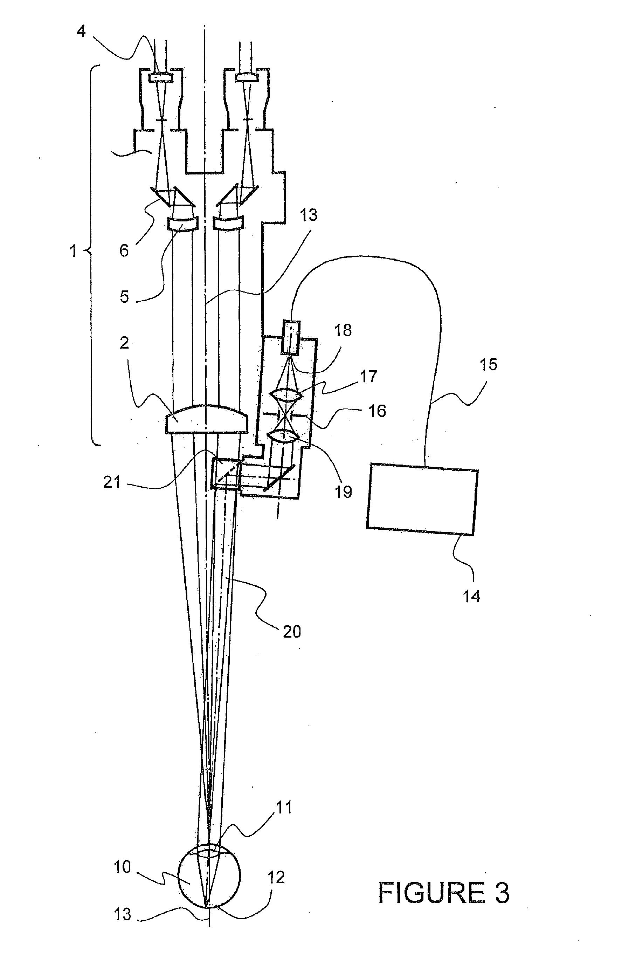

Enfin dans le mode de réalisation de la figure 3, le prisme 21 est placé entre l'objectif principal 2 et l'oeil 10, c'est-à-dire à l'extérieur d'un microscope traditionnel.

Le prisme 21 est donc alors semi-réfléchissant, traversé par le faisceau d'observation.

Ce dernier mode de réalisation est très avantageux car il permet de constituer la fente 16 et les éléments optiques d'éclairage qui lui sont associés, c'est-à-dire l'extrémité 18 du faisceau de fibres, le condenseur 17, le collimateur 19 et le prisme 21 en un ensemble autonome qui peut être rapporté par l'intermédiaire d'une fixation autour du corps de l'objectif principal d'un microscope traditionnel. Dans ce mode de réalisation, le prisme 21 peut soit être placé en dehors du faisceau d'observation et il sera alors avantageusement un miroir, soit placé dans le faisceau d'observation, il devra alors être semi-transparent.

On comprend qu'il est ainsi possible de faire bénéficier des avantages de la présente invention, l'ensemble des microscopes pour la microchirurgie oculaire déjà existant, en fournissant cet ensemble autonome qui peut être rapporté a posteriori sur ces appareils.

Quel que soit le mode de réalisation concerné, la largeur de la fente est avantageusement réglable permettant aux praticiens de faire l'observation qu'ils recherchent dans les meilleures conditions. Il est également possible de doter l'appareil de l'invention d'un barillet portant plusieurs fentes, le praticien choisissant celle d'entre elles qui est la mieux adaptée.

## Revendications

1. Microscope pour la microchirurgie oculaire comprenant :
- un objectif principal (2),
- au moins un système oculaire (3), et
- des moyens d'éclairage à fente,
l'image de la fente (16) étant projetée sur le fond d'oeil (10) au travers d'un prisme (21) placé à proximité de la direction d'observation du microscope,
**caractérisé en ce que** le prisme (21) est relié à la source lumineuse (14) par un faisceau (15) de fibres optiques, ladite source (14) étant indépendante du microscope.

2. Microscope pour la chirurgie oculaire selon la revendication 1, **caractérisé en ce que** les moyens d'éclairage à fente produisent un faisceau d'éclairage (20) incliné par rapport à la direction d'observation (13) d'un angle fixe.

3. Microscope pour la chirurgie oculaire selon la revendication 2, **caractérisé en ce que** les moyens d'éclairage à fente produisent un faisceau d'éclairage (20) incliné par rapport à la direction d'observation (13) d'un angle compris entre 2 et 10 degrés.

4. Microscope pour la microchirurgie oculaire selon l'une des revendications 1 à 3, **caractérisé en ce que** le prisme (21) est placé en amont de l'objectif principal (2).

5. Microscope pour la microchirurgie oculaire selon la revendication 4, **caractérisé en ce que** le prisme (21) est placé en amont d'une partie au moins des éléments optiques (7) de l'un des systèmes oculaires.

6. Microscope pour la microchirurgie oculaire selon l'une quelconque des revendications 1 à 3, **caractérisé en ce que** le prisme (21) est placé en aval de l'objectif principal (2) grâce à une fixation rapportée autour du corps de l'objectif principal (2).

7. Microscope pour la microchirurgie oculaire selon l'une quelconque des revendications 1 à 6, **caractérisé en ce que** l'éclairage de la lampe à fente est indépendant d'un éclairage coaxial du microscope.

8. Microscope pour la microchirurgie oculaire selon l'une quelconque des revendications 1 à 7, **caractérisé en ce que** la largeur de la fente (16) est réglable.

9. Microscope pour la microchirurgie oculaire selon l'une quelconque des revendications 1 à 8, **caractérisé en ce que** la fente (16) est portée par un barillet en comportant plusieurs de largeurs différentes permettant au praticien de choisir l'une d'entre elles.
